# EUROPÄISCHE PATENTANMELDUNG

(11) **EP 4 606 290 A1**
(43) Veröffentlichungstag der Anmeldung: **27.08.2025**
(21) Anmeldenummer: 24159116.3
(22) Anmeldetag: 22.02.2024
(51) Int. Cl.: A61B 1/00, G16H 40/63, A61B 34/20, A61B 34/00, A61B 34/30, A61B 90/00

(54) **OPERATIONSEINRICHTUNG UND VERFAHREN ZUM EINSTELLEN EINES MEDIZINISCHEN GERÄTS**

(71) Anmelder: Erbe Elektromedizin GmbH, 72072 Tübingen (DE)
(72) Erfinder: JURJUT, Ovidiu, 64625 Bensheim (DE); KALIM, Faisal, 72764 Reutlingen (DE); JOSHI, Shirish, 78573 Wurmlingen (DE)
(74) Vertreter: Rüger Abel Patentanwälte PartGmbB

(57) **Zusammenfassung**

Die erfindungsgemäße Einrichtung (10) weist eine Bildaufnahmeeinrichtung (14) auf, an die eine Bildauswerteeinrichtung (25) und über diese eine Bildwiedergabeeinrichtung (18) angeschlossen ist. Die Bildverarbeitungseinrichtung (25) ist darauf eingerichtet, ein Menü (20) in das Bild der Bildverarbeitungseinrichtung (18) einzublenden. Das Einblenden des Menüs (20) kann durch eine geeignete Bewegung des Instruments (11) ausgelöst werden. Beispielsweise kann dazu am Bildrand ein Aktivierungsbereich (A) vorgesehen sein, der von der Bildverarbeitungseinrichtung (25) darauf überwacht wird, ob die Instrumentenspitze (19) diesen Bereich berührt. Ist dies der Fall, kann ein entsprechendes diese Gestik kennzeichnendes Aktivierungssignal (a) erzeugt und zur Einblendung des Menüs (20) in das Bild verwendet werden. Weitere Bewegungen der Instrumentenspitze (19), insbesondere in einem festgelegten Erfassungsgebiet (E), ermöglicht die Steuerung eines Zeigers (21) in dem Menü (20) und die Auswahl von Funktionen zur Steuerung des Geräts (15).

## Beschreibung

Die Erfindung betrifft eine Einrichtung zur Behandlung des Körpers eines menschlichen oder tierischen Patienten. Insbesondere betrifft die Erfindung eine Einrichtung, bei der ein das behandelnde Instrument speisendes Gerät durch Manipulation des Instruments einstellbar ist, sowie ein Verfahren dazu.

Aus der WO 2022/216810 A2 sind ein System, ein Verfahren und eine Einrichtung zur Verfolgung eines Instruments über ein digitales Mikroskop bekannt. Dieses erzeugt ein Bild und ermöglicht die Bedienung einer in das Bild eingeblendeten graphischen Nutzerschnittstelle mittels der Spitze des Instruments, die von einer Bildanalyseeinrichtung erkannt werden kann. Die in das Bild eingeblendete graphische Nutzerschnittstelle wird vorzugsweise im Randbereich des Bildes außerhalb des interessierenden Operationsbereichs dargestellt. Bedarfsweise kann die Nutzerschnittstelle auch ausgeblendet werden. Sobald der Nutzer die Instrumentenspitze in den Bereich der graphischen Nutzerschnittstelle bewegt, kann diese angezeigt werden. Danach können entsprechende Felder der graphischen Nutzeroberfläche ausgewählt werden, indem die Instrumentenspitze eine Mindestzeit in diesem Bereich gehalten wird.

Zur Bedienung der graphischen Nutzerschnittstelle muss der Bediener mit seiner Instrumentenspitze die am Bildrand liegende graphische Nutzerschnittstelle aufsuchen und die Instrumentenspitze dort für die Dauer der Bedienung belassen.

Es ist Aufgabe der Erfindung, eine Einrichtung anzugeben, die eine alternative Bedienung einer graphischen Nutzerschnittstelle mittels eines Instruments gestattet. Weiter ist es Aufgabe der Erfindung, ein Verfahren zur Bedienung der graphischen Nutzerschnittstelle anzugeben.

Diese Aufgaben werden mit der Einrichtung nach Anspruch 1 und dem Verfahren nach Anspruch 15 gelöst:

Die erfindungsgemäße Einrichtung umfasst eine Bildaufnahmeeinrichtung, die dazu eingerichtet ist, ein Bild von einem Operationsort aufzunehmen und an eine Bildverarbeitungseinrichtung zu übertragen. Die Bildaufnahmeeinrichtung kann eine Endoskopkamera oder eine sonstige Kamera sein. Alternativ kann ein mit einem oder mehreren Arbeitskanälen versehenes Endoskop vorgesehen sein, das an seinem distalen Ende eine Kamera zur Aufnahme von Bildern oder Videostreams von einem Operationsort aufweist.

Zu der Einrichtung gehört weiter ein Instrument, dessen Instrumentenspitze in das Sichtfeld der Bildaufnahmeeinrichtung hinein bewegbar ist. Ein solches Instrument kann beispielsweise ein von der Bildaufnahmeeinrichtung gesondertes und unabhängig von dieser geführtes Instrument sein. Es kann sich auch um ein als Sonde ausgebildetes steifes oder flexibles Instrument handeln, das durch einen Arbeitskanal eines Endoskops in den Sichtbereich der Bildaufnahmeeinrichtung bewegbar und somit positionierbar ist. Zur Positionierung der Instrumentenspitze im Bildbereich kann eine Positioniereinrichtung dienen, mittels derer die Instrumentenspitze quer zu seiner Längsrichtung bewegbar ist. Dazu können in oder an dem Instrument Mittel vorgesehen sein, mittels derer sich die Instrumentenspitze abwinkeln lässt. Ist das Instrument ein laparoskopisches Instrument mit einem steifen Schaft, ist der am proximalen Ende vorgesehene Griff das Positionierungsmittel, durch das das distale Ende positionierbar ist.

An die Bildverarbeitungseinrichtung ist eine Bildwiedergabeeinrichtung angeschlossen, die dazu eingerichtet ist, das von der Bildaufnahmeeinrichtung aufgenommene Bild wiederzugeben. Die Bildwiedergabeeinrichtung kann beispielsweise ein Monitor, eine VR-Brille oder dergleichen sein. Die Bildwiedergabeeinrichtung kann in mittelbarer Nähe des Operationsorts oder, z.B. bei Fernoperationen, auch in größerer Distanz zu diesem angeordnet sein.

Die Bildwiedergabeeinrichtung und die Bildverarbeitungseinrichtung sind dazu eingerichtet, in dem wiedergegebenen Bild bedarfsweise ein Menü anzuzeigen, das eine graphische Benutzerschnittstelle darstellt. Vorzugsweise ist dieses Menü außerhalb des Zentrums des aufgenommenen Bilds angeordnet, um die typischerweise im Zentrum des aufgenommenen Bilds liegende interessierende und der Operation unterliegende Region menschlichen oder tierischen Gewebes nicht zu verdecken. Das außerhalb des Zentrums eingeblendeten Menü enthält ein Zeigersymbol. Vorzugsweise ist dies immer vorhanden. Weiter vorzugsweise ist das Zeigersymbol nur innerhalb des Menüs und nicht über dessen Rand hinaus bewegbar.

Die Bildverarbeitungseinrichtung ist darauf eingerichtet, das Instrument, insbesondere seine Instrumentenspitze, zu erfassen und mit dem Zeigersymbol Bewegungen der Instrumentenspitze nachzuvollziehen. Das Zeigersymbol und die Instrumentenspitze sind somit bewegungsgekoppelt. Das Zeigersymbol wird dabei in einer Distanz zu der Instrumentenspitze wiedergegeben. Damit kann die Instrumentenspitze bei einer Bedienung des Menüs, d.h. der graphischen Nutzerschnittstelle im Bildzentrum, d.h. im Bereich des Operationsorts verbleiben und dennoch zur Bedienung des Menüs herangezogen werden. Dies ist insbesondere dann von Vorteil, wenn eine größere Bewegung der Instrumentenspitze aus chirurgischer Sicht vermieden werden soll oder die Region des eingeblendeten Menüs nur schwer erreichbar ist. Bei einer bevorzugten Ausführungsform sind die Bildwiedergabeeinrichtung und/oder die Bildverarbeitungseinrichtung darauf eingerichtet, in dem Bild ein Erfassungsgebiet festzulegen, das außerhalb des Menüs angeordnet ist. Weiter können die Bildwiedergabeeinrichtung und/oder die Bildverarbeitungseinrichtung darauf eingerichtet sein, die Instrumentenspitze und deren Bewegungen nur dann zur Bewegung und Positionierung des Zeigers zu nutzen, wenn sich die Instrumentenspitze in dem Erfassungsgebiet befindet.

Vorzugsweise ist für das auf der Bildwiedergabeeinrichtung sichtbare Bild eine Randzone definiert, wobei die Bildwiedergabeeinrichtung und/oder die Bildverarbeitungseinrichtung überwacht, ob die Instrumentenspitze in die Randzone positioniert worden ist. Die Bildwiedergabeeinrichtung und/oder die Bildverarbeitungseinrichtung kann darauf eingerichtet sein, das normalerweise unsichtbare Menü in dem auf der Bildwiedergabeeinrichtung wiedergegebenen Bild sichtbar zu machen, sobald die Instrumentenspitze in der Randzone positioniert ist und/oder dort längere Zeit verbleibt.

Nach Aufblendung des Menüs kann mit der Instrumentenspitze ein in dem Menü sichtbares Zeigersymbol bewegt werden, um einzelne Menüfelder auszuwählen. Dazu kann die Bildverarbeitungseinrichtung insbesondere darauf eingerichtet sein, das Zeigersymbol entsprechend der Bewegung der Instrumentenspitze zu bewegen, sobald eine Bewegung der Instrumentenspitze von der Bildverarbeitungseinrichtung erfasst wird. Dabei kann die Bildverarbeitungseinrichtung auch darauf eingerichtet sein, eine Bewegung der Instrumentenspitze nur dann zur Bewegung des Zeigersymbols heranzuziehen, wenn die Instrumentenspitze in dem dafür vorgesehenen, zum Beispiel zentral angeordneten Erfassungsgebiet positioniert ist. Dieses Erfassungsgebiet kann vorzugsweise im Zentrum des Bilds angeordnet sein, indem sich die Instrumentenspitze bei Durchführung einer Operation ohnehin befindet.

Die einzelnen Menüfelder können unterschiedlichen technischen Aktionen zugeordnet sein, wie insbesondere der Auswahl oder der Veränderung von Einstellungen eines Generators oder sonstig das Instrument speisenden Geräts. Zum Beispiel können die Menüfelder unterschiedlichen Modes und/oder unterschiedlichen Effektstärken zugeordnet sein. Das Menü kann Untermenüs aufweisen, um beispielsweise ausgewählte Modes in ihrer Effektstärke verändern zu können.

Die Bewegung der Instrumentenspitze kann proportional (d.h. mit gleichbleibendem Abstand zu dem Zeigersymbol) sowie bedarfsweise auch mit einem Vergrößerungsfaktor oder mit einem Verkleinerungsfaktor auf die Bewegung des Zeigers übertragen werden, mit dem der von dem Zeiger zugrückgelegte Weg gegenüber dem von der Instrumentenspitze zurückgelegten Weg entsprechend vergrößert oder verkleinert wird. So kann der vom Zeiger bei einer Bewegung der Instrumentenspitze zurückgelegte Weg größer oder auch kleiner als der von der Instrumentenspitze zurückgelegte Weg sein. Jedenfalls aber wird es bevorzugt, dass die Richtungen der Wege der Instrumentenspitze und des Zeigers zu jedem Zeitpunkt in dem Bild miteinander übereinstimmen.

Die erfindungsgemäße Einrichtung weist außerdem eine Quittiereinrichtung auf, die dazu dient, dasjenige Menüfeld auszuwählen oder zu aktivieren, in dem sich der Zeiger befindet. Die Quittiereinrichtung kann ein Fußschalter, ein Handschalter oder auch eine Gestenerkennungseinrichtung sein, die Teil der Bildverarbeitungseinrichtung sein kann. Zum Beispiel kann die zur Auslösung eines Quittiersignals dienende Geste eine stechende Bewegung der Instrumentenspitze sein. Auch kann eine Kreisbewegung der Instrumentenspitze als Quittiersignal erfasst werden. Auch können andere Gesten zur Generierung eines Quittiersignals vorgesehen sein. Z.B. kann eine Auswahl von Menüfeldern durch eine Vertikalbewegung des Zeigers und eine Bestätigung (d.h. Quittierung) eines Menüfeldes durch eine Horizontalbewegung des Zeigers innerhalb des Menüfeldes erfolgen.

Bei der bevorzugten Ausführungsform wird ein Aktivierungssignal zum Einblenden eines Menüs erzeugt, indem mit der Instrumentenspitze ein Aktivierungsgebiet berührt wird. Die Bedienung des Menüs erfolgt vorzugsweise durch die Instrumentenspitze erst, wenn sich diese in dem Erfassungsgebiet befindet. Ein Quittiersignal zur Bestätigung eines ausgewählten Menüfelds kann erzeugt werden, wenn die Instrumentenspitze in dem Erfassungsgebiet eine vorfestgelegte Geste, zum Beispiel eine stechende Bewegung, ausführt.

Mit der erfindungsgemäßen Einrichtung und dem entsprechenden erfindungsgemäßen Verfahren ist eine erleichterte Bedienung eines Generators durch den Operateur möglich, ohne dass dieser den Blick von dem Operationsort wenden und ohne dass dieser den Operationsort nachhaltig verlassen muss.

Weitere Einzelheiten und Vorteile einer erfindungsgemäßen Ausführungsform der Erfindung sowie von Abwandlungen derselben ergibt sich aus der nachfolgenden Beschreibung, der Zeichnung. In dieser zeigen:
Figur 1 die erfindungsgemäße Einrichtung, in Übersichtsdarstellung,
Figur 2 bis 7 ein von einer Bildwiedergabeeinrichtung wiedergegebenes Bild und die Spitze des darin sichtbaren Instruments in verschiedenen Bedienstadien, in schematisierter Darstellung.

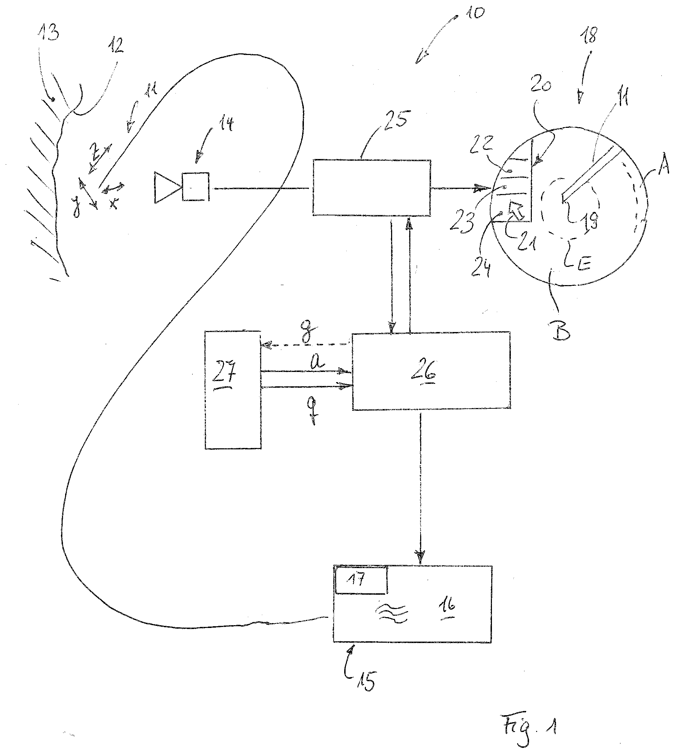

Aus Figur 1 ist eine Einrichtung 10 zur chirurgischen Behandlung eines Patienten bekannt, der in Figur 1 lediglich anhand einer durch ein Instrument 11 zu behandelnden Gewebefläche 12 veranschaulicht ist. Die Gewebefläche 12 ist ein Operationsort und kann zum Beispiel die innere Oberfläche eines Hohlorgans 13, wie beispielsweise des Magens, des Darms oder anderer Organe oder Gewebepartien sein. Bei der Darstellung nach Figur 1 wird davon ausgegangen, dass die Operation endoskopisch durchgeführt wird, wobei auf die Darstellung des Endoskops verzichtet ist. Es sind deswegen lediglich das zum Beispiel als flexible Sonde ausgebildete Instrument 11 sowie eine Bildaufnahmeeinrichtung 14 veranschaulicht, bei der es sich beispielsweise um eine Endoskopkamera handeln kann.

Das Instrument 11 kann starr, z.B. als laparoskopische Instrument, oder flexibel ausgebildet sein, z.B. als Endoskopsonde. Es wird zur Durchführung einer Operation mit der Gewebefläche 12 in Wechselwirkung oder zumindest in die Nähe derselben gebracht und entsprechend den Erfordernissen der Durchführung der Operation bewegt. Bewegungsrichtungen sind in Figur 1 durch Pfeile symbolisiert, die Bewegungsrichtungen x, y, z symbolisieren. Das als Sonde ausgebildete Instrument 11 kann durch einen Arbeitskanal eines Endoskops geführt sein, wobei das Endoskop eine Bildaufnahmeeinrichtung 14 aufweisen kann. Das Instrument 11 kann zum Beispiel auch starr ausgebildet sein, wobei an dem proximalen Ende Ein Handgriff zur Positionierung bei des starren Instruments 11 innerhalb des Patienten vorgesehen ist. Bei laparoskopischen Eingriffen kann die die Bildaufnahmeeinrichtung 14 getrennt und unabhängig von dem Instrument 11 in den Körper des Patienten eingeführt sein. Alle zum Bewegen des (flexiblen oder starren) Instruments 11 vorgesehenen Elemente bilden zusammen die Positioniereinrichtung für das Instrument 11.

Das Instrument 11 ist beispielsweise ein HF-chirurgisches Instrument, das dazu mit einem speisenden Gerät 15, beispielsweise in Gestalt eines HF-Generators 16, verbunden ist. Jedoch sind auch andere Anwendungen möglich. Beispielsweise kann das Gerät 15 ein Gerät sein, das zusätzlich oder alternativ zur HF-Erzeugung zur Gasversorgung des Instruments 11, zur Spülung des Operationsorts, zur Gasabsaugung oder zu anderen Zwecken eingerichtet ist.

Zur Einstellung des Geräts 16 kann dieses eine ganze Anzahl von in Figur 1 lediglich symbolisch dargestellten Einstellmitteln 17, wie zum Beispiel Tasten, Drehknöpfen, Bildschirme und dergleichen aufweisen. Die Bedienung derselben erfordert aber die Hinwendung zu den Gerät 16 entweder durch den Operateur selbst, der das Instrument 11 führt, oder durch eine weitere Bedienperson. Die Aufmerksamkeit des Operateurs richtet sich jedoch vorzugsweise auf eine Wiedergabeeinrichtung 18, die das von der Bildaufnahmeeinrichtung 14 aufgenommene Bild B zeigt. In diesem ist das distale Ende des Instruments 11 mit seiner Instrumentenspitze 19 sichtbar und im Sichtfeld der Bildaufnahmeeinrichtung 14 und somit in dem von der Wiedergabeeinrichtung 18 wiedergegebenen Bild B bewegbar.

Bedarfsweise können in das Bild B graphische Elemente, wie zum Beispiel ein Menü 20 und ein in dem Menü 20 vorhandener Zeiger 21, eingeblendet sein. Der Zeiger 21 ist ein grafisches Symbol, z.B. ein Punkt oder ein Kreis oder ein Dreieck oder ein Pfeil oder das Symbol einer Hand oder ein sonstiges Symbol. Das Menü 20 kann mehrere Menüfelder 22, 23, 24 umfassen, wobei der Zeiger 21 über diese Menüfelder 20, 23, 24 bewegbar ist. Den Menüfeldern 22, 23, 24 können Funktionen zugeordnet sein, wie beispielsweise das Ein- und Ausschalten von Funktionen des Geräts 15, die Beeinflussung von Effektstärken und/oder das Aufblenden von Untermenüs zur weiteren Befehlsgabe. Die Menüfelder 22, 23, 24 können rechteckige oder anderweitig geformte Flächen sein, die mit Kennfarben, Symbolen oder Beschriftungen versehen sein können, um die Funktionen zu verdeutlichen, die über sie auswählbar sind. Vorzugsweise ist der Zeiger 21 durchscheinend dargestellt, so dass die Funktion eines Menüfelds 22, 23, 24 sicht- und erkennbar bleibt, wenn der Zeiger 21 auf dem betreffenden Menüfeld steht.

Zwischen der Bildaufnahmeeinrichtung 14 und der Bildwiedergabeeinrichtung 18 ist eine Bildverarbeitungseinrichtung 25 angeordnet, die dazu eingerichtet ist, die Instrumentenspitze 19 in dem von der Bildaufnahmeeinrichtung 14 aufgenommenen Bild zu lokalisieren und anhand dessen das Gerät 15 zu steuern. Dazu ist die Bildverarbeitungseinrichtung 25 mit einer Steuereinrichtung 26 verbunden, die dazu eingerichtet ist, Steuerbefehle für das Gerät 15 zu erzeugen und an dieses zu übermitteln. Die Steuereinrichtung 26 kann außerdem mit einer Quittiereinrichtung 27 verbunden sein, die dazu vorgesehen sein kann, Aktivierungssignale a zu erzeugen, um die Steuereinrichtung 26 und die Bildverarbeitungseinrichtung 25 zu veranlassen, das Menü 20 in das Bild einzublenden. Außerdem kann die Quittiereinrichtung 27 dazu eingerichtet sein, Quittiersignale q zu erzeugen, um Menüfelder 22, 23 oder 24 zu aktivieren, auf denen der Zeiger 21 steht. Bei Empfang eines Quittiersignals q gibt die Steuereinrichtung 26 ein dem betreffenden Menüfeld 22, 23 oder 24 entsprechendes Steuersignal an das Gerät 15.

Die Quittiereinrichtung 27 kann eine manuelle Quittiereinrichtung zum Beispiel in Gestalt eines von Hand oder Fuß bedienbaren Tasters sein. Es kann sich außerdem um eine Einrichtung zur Erkennung von Gesten der Instrumentenspitze 19 handeln. Die Erkennung solcher Gesten kann von der Bildverarbeitungseinrichtung 25 und/oder der Steuereinrichtung 26 vorgenommen werden. Beispielsweise kann die Bildverarbeitungseinrichtung 25 entsprechende Gesten g erkennen und als entsprechende Signale an die Quittiereinrichtung 27 übermitteln, die daraufhin entsprechend Aktivierungssignale a oder Quittiersignale q generiert und an die Steuereinrichtung 26 übermittelt. Alternativ kann die Bildverarbeitungseinrichtung 25 darauf eingerichtet sein, die Gesten zu erkennen und ein entsprechendes Signal g an die Quittiereinrichtung 27 oder direkt an die Steuereinrichtung 26 zu senden. Die Auswahl eines Menüfeldes z.B. durch eine Bewegung des Zeigers 21 entlang der Reihe der Menüfelder 22, 23, 24, hier z.B. in Form einer Vertikalbewegung erfolgen. Eine Geste zur Bestätigung (d.h. Quittierung) eines Menüfeldes (z.B. Menüfeld 23) und zur Erzeugung eines Quittiersignals q kann z.B. eine Horizontalbewegung des Zeigers 21 innerhalb des betreffenden Menüfeldes 23 sein.

Die insoweit beschriebene Einrichtung 10 arbeitet wie folgt:

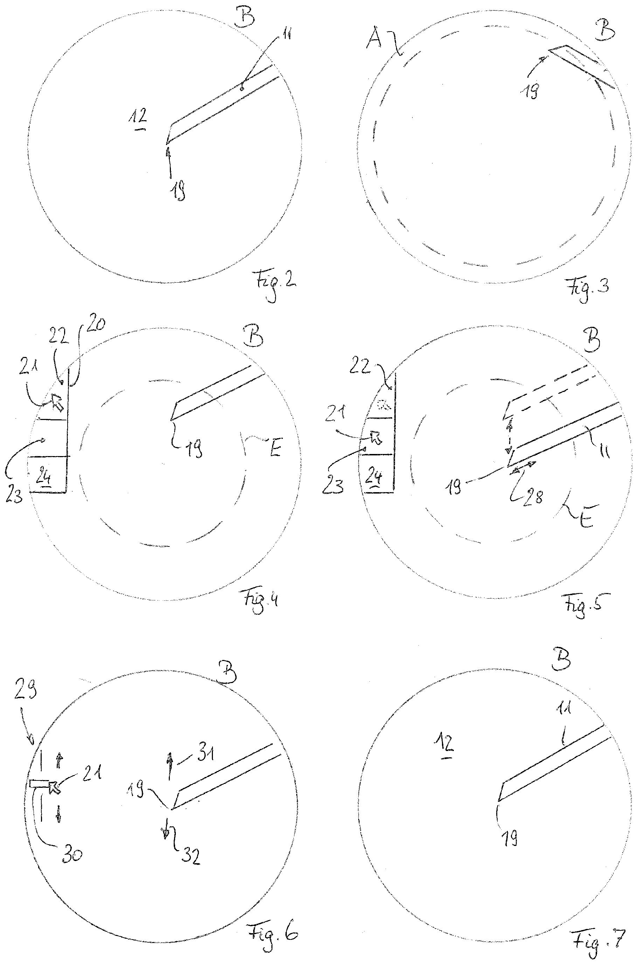

Figur 2 veranschaulicht zunächst das von der Bildaufnahmeeinrichtung 14 aufgenommene Bild B mit darin sichtbarer Instrumentenspitze 19. Diese ist etwa im Zentrum des Bildes und somit in dem typischerweise interessierenden Bereich des Gewebes 12 abgebildet, auf das mittels des Instruments 11 Einfluss genommen werden soll.

Es wird nun davon ausgegangen, dass der Bediener eine Einstellung des Geräts 15 vornehmen will ohne seinen Blick von dem Bild B zu wenden. Deswegen bewegt er die Instrumentenspitze 19 nun in einen Aktivierungsbereich A hinein, der beispielsweise der gesamte ringförmige Randbereich des Bildes B sein kann. Sobald die Instrumentenspitze 19, wie in Figur 3 veranschaulicht, diesen Aktivierungsbereich A berührt, erfasst die Bildverarbeitungseinrichtung 25 oder die Steuereinrichtung 26 dies als entsprechende Geste g und sendet ein entsprechendes Signal an die Quittiereinrichtung 27. Diese generiert ein Aktivierungssignal a, das entweder auf den Umweg über die Steuereinrichtung 26 oder auch auf direktem Weg die Bildverarbeitungseinrichtung 25 dazu veranlasst, das Menü 20 in das Bild B einzublenden wie in Figur 4 sichtbar ist.

Mit der Einblendung des Menüs 20 in das Bild B nach Figur 4 kann ein Erfassungsgebiet E festgelegt werden, das ein Teil des Bilds B, beispielsweise einen mittleren Bereich, oder alternativ auch das gesamte Bild B einnimmt. Dieses Erfassungsgebiet E wird von der Bildverarbeitungseinrichtung 25 auf Bewegungen der Instrumentenspitze 19 überwacht, sobald sich diese in dem Erfassungsgebiet E befindet. Von der Bildverarbeitungseinrichtung 25 erfasste Bewegungen der Instrumentenspitze 19 werden entsprechend auf Bewegungen des Zeigers 21 übertragen.

Letzteres zeigt der Zusammenhang von Figur 4 und Figur 5. Wird die Instrumentenspitze 19 beispielsweise von der Position gemäß Figur 4 in die Position gemäß Figur 5 bewegt, was in Figur 5 durch einen gestrichelten Pfeil veranschaulicht ist, wird der Zeiger 21 aus der in Figur 5 ersichtlichen gestrichelten vormaligen Position heraus und von dem Menüfeld 22 in das Menüfeld 23 bewegt. Die Richtung der Bewegung des Zeigers 21 stimmt dabei mit der Richtung der Bewegung der Instrumentenspitze 19 überein. Auf diese Weise kann der Zeiger 21 über die verschiedenen Menüfelder 22, 23, 24 bewegt werden, um eines dieser Menüfelder 22 bis 24 auszuwählen. Vorzugsweise ist der Bewegungsbereich des Zeigers 21 auf das Menü 20 beschränkt, d.h. der Zeiger 21 kann das Menü 20 nicht verlassen.

Die Auswahl eines Menüfelds 22, 23, oder 24 erfolgt dann durch ein Quittiersignal q. Dieses kann mittels der Quittierseinrichtung 22 generiert und an die Steuereinrichtung 26 übergeben werden. Zur Erzeugung des Quittiersignals q kann ein Taster, zum Beispiel an dem Instrument, ein Fußschalter oder auch die Erkennung einer Geste dienen. Beispielsweise kann gemäß Figur 5 mit dem Instrument 11 eine stechende Geste durchgeführt werden, die in Figur 5 durch einen Pfeil 28 angedeutet ist. Die stechende Geste kann eine kurze schnelle Bewegung des Instruments in Instrumentenlängsrichtung sein. Findet diese Geste in dem Erfassungsgebiet E statt, kann dies erkannt und als Quittiersignal q an die Steuereinrichtung 26 geliefert werden. Diese kann daraufhin, je nach Bedeutung des ausgewählten Felds, hier das Menüfeld 21, den Generator 16 (oder ein sonstiges Gerät 15) in den dem Menüfeld 23 zugeordneten Mode überführen, beispielsweise Koagulation.

Falls gewünscht, kann die Bildverarbeitungseinrichtung 25 ein weiteres Menüfeld 29 gemäß Figur 6 in das Bild B einblenden, um beispielsweise die Stärke des gewünschten Effekts einstellen zu können. Der Zeiger 21 kann dabei einen symbolischen als Grafik veranschaulichten Schieber 30 auf und ab bewegen, was durch eine entsprechende Bewegung der Instrumentenspitze 19 in Richtung der Pfeile 31, 32 bewirkt werden kann. Nach Verstreichen einer vorgewählten Zeit oder alternativ durch eine entsprechende Geste, beispielsweise wiederum die stechende Geste gemäß Pfeil 28 in Figur 5, kann das Menüfeld 29 wieder ausgeblendet werden, wonach das Bild gemäß Figur 7 mit dem Instrument 11 darin ohne weitere graphische Einblendungen sichtbar ist. Das Instrument 11 steht nun mit den gewählten Einstellungen zur Behandlung der Gewebefläche 12 bereit.

Bei der vorigen Beschreibung ist davon ausgegangen worden, dass die Bewegung der Instrumentenspitze 19, insbesondere in vertikaler Richtung, 1:1 auf den Zeiger 21 übertragen wird. Es ist jedoch auch möglich, den von der Instrumentenspitze 19 zurückgelegten Weg vergrößert auf den Zeiger 21 zu übertragen, wie beispielsweise um die Bedienung des Menüs 20 mit minimalen Instrumentenbewegungen bewirken zu können. Alternativ kann der Weg des Zeigers 21 auch eine proportional verkleinerte Version des von der Instrumentenspitze 19 zurückgelegten Wegs sein, beispielsweise um eine besonders feinfühlige Menübedienung zu ermöglichen. Insbesondere ist es möglich, unterschiedliche Übertragungsmaßstäbe der Instrumentenspitze 19 zu dem Zeiger 21 für das Hauptmenü 20 und das Menüfeld 29 festzulegen. Weiter ist es möglich, anstelle der Instrumentenspitze 19 einen anderen ausgewählten Punkt des Instruments 11 zur Steuerung des Menüs 20 und des Zeigers 21 zu nutzen. Die vorliegende Beschreibung der Erfindung und ihrer Ausführungsformen gilt dann entsprechend mit der Maßgabe, dass die ausgewählte Stelle des Instruments 11 an die Stelle der Instrumentenspitze 19 tritt.

Weiter ist es möglich, lediglich die Vertikalbewegung der Instrumentenspitze 19 auf dem Zeiger 21 zu übertragen, während eine Horizontalbewegung der Instrumentenspitze 19 nicht auf den Zeiger 21 übertragen wird. Auf diese Weise wird die Bedienung des Menüs 20 erleichtert und es werden Fehlbedienungen, insbesondere das Herausführen des Zeigers 21 aus dem Menü 20 oder das Hängenbleiben desselben am Rand des Menüs, vermieden. Die Bildverarbeitungseinrichtung 25 muss nicht, aber kann darauf eingerichtet sein, die Beweglichkeit des Zeigers 21 auf das Menü 20 zu beschränken. Auch dies erleichtert die Bedienung und ermöglicht es insbesondere, den Zeiger 21 an derjenigen Stelle des Erfassungsgebiets E mit der Instrumentenspitze 19 zu bedienen, in der sich das Instrument 11 am besten bewegen lässt.

Die erfindungsgemäße Einrichtung 10 weist eine Bildaufnahmeeinrichtung 14 auf, an die eine Bildauswerteeinrichtung 25 und über diese eine Bildwiedergabeeinrichtung 18 angeschlossen ist. Die Bildverarbeitungseinrichtung 25 ist darauf eingerichtet, ein Menü 20 in das Bild B der Bildverarbeitungseinrichtung 18 einzublenden. Das Einblenden des Menüs 20 kann durch eine geeignete Bewegung des Instruments 11 ausgelöst werden. Beispielsweise kann dazu am Bildrand ein Aktivierungsbereich A vorgesehen sein, der von der Bildverarbeitungseinrichtung 25 darauf überwacht wird, ob die Instrumentenspitze 19 diesen Bereich berührt. Ist dies der Fall, kann ein entsprechendes diese Gestik kennzeichnendes Aktivierungssignal a erzeugt und zur Einblendung des Menüs 20 in das Bild verwendet werden. Weitere Bewegungen der Instrumentenspitze 19, insbesondere in einem festgelegten Erfassungsgebiet E, ermöglicht die Steuerung eines Zeigers 21 in dem Menü 20 und die Auswahl von Funktionen zur Steuerung des Geräts 15.

### Bezugszeichen:

- 10: Einrichtung zur chirurgischen Behandlung eines Patienten
- 11: Instrument
- 12: Gewebefläche
- 13: Hohlorgan
- 14: Bildaufnahmeeinrichtung
- X, y, z: Bewegungsrichtungen
- 15: Gerät zur Speisung des Instruments 11
- 16: Generator
- 17: Bedienelemente
- 18: Bildwiedergabeeinrichtung
- B: Bild auf der Bildwiedergabeeinrichtung 18
- 19: Instrumentenspitze
- 20: Menü
- 21: Zeiger
- 22 - 24: Menüfelder
- 25: Bildverarbeitungseinrichtung
- 26: Steuereinrichtung / Erfassungseinrichtung
- 27: Quittiereinrichtung
- A: Aktivierungsbereich
- E: Erfassungsgebiet
- 28: Pfeil
- 29: Menüfeld zur Effektstärkeeinstellung
- 30, 31: Pfeile

## Patentansprüche

1. Einrichtung (10) zur chirurgischen Behandlung eines menschlichen oder tierischen Patienten,
mit einer Bildaufnahmeeinrichtung (14), die dazu eingerichtet ist, ein Bild (B) von einem Operationsort (12) aufzunehmen und an eine Bildverarbeitungseinrichtung (25) zu übertragen,
mit einem Instrument (11), das zur Einwirkung auf den Patienten an dem Operationsort (12) eingerichtet und innerhalb dessen beweglich führbar ist,
mit einer Bildwiedergabeeinrichtung (18), die an die Bildverarbeitungseinrichtung (25) angeschlossen und dazu eingerichtet ist,
a) sowohl das vom Operationsort (12) aufgenommene Bild (B) sowie, bedarfsweise,
b) außerhalb des Zentrums des aufgenommenen Bilds (B) wenigstens ein Menü (20) und
c) ein Zeigersymbol (21) anzuzeigen, das mit dem Instrument (11) bewegungsgekoppelt ist.

2. Einrichtung nach Anspruch 1, **dadurch gekennzeichnet, dass** das Instrument (11) eine distale in dem Operationsort (12) positionierbare Instrumentenspitze (19) aufweist.

3. Einrichtung nach Anspruch 2, **dadurch gekennzeichnet, dass** dem Instrument (11) eine Positioniereinrichtung zugeordnet ist, die dazu eingerichtet ist, seine Instrumentenspitze (19) in dem von der Bildaufnahmeeinrichtung (14) aufgenommenen Bild (B) in zwei voneinander unabhängigen Richtungen (x, y) zu bewegen.

4. Einrichtung nach einem der Ansprüche 2 oder 3,
**dadurch gekennzeichnet, dass** der Zeiger (21) mit der Instrumentenspitze (19) bewegungsgekoppelt ist.

5. Einrichtung nach Anspruch 4, **dadurch gekennzeichnet, dass** das Zeigersymbol (21) in wenigstens einer Richtung (x, y) von der Instrumentenspitze () beabstandet in dem Bild (B) positioniert ist.

6. Einrichtung nach einem der vorstehenden Ansprüche,
**dadurch gekennzeichnet, dass** das Menü (20) mehrere Menüfelder (22, 23, 24, 29) umfasst, denen unterschiedliche technische Aktionen zugeordnet sind, und dass die technischen Aktionen die Auswahl oder Veränderung von Einstellungen eines Geräts (15) sind, die zu unterschiedlichen Modes und Effektstärken gehören.

7. Einrichtung nach einem der Ansprüche 2 bis 4, **dadurch gekennzeichnet, dass** zu der Einrichtung (10) eine mit der Bildverarbeitungseinrichtung (25) verbundene Erfassungseinrichtung (26) gehört, die darauf eingerichtet ist, die Position der Instrumentenspitze (19) in dem Bild (B) zu erfassen und an die Bildverarbeitungseinrichtung (25) zu übermitteln, wobei die Bildverarbeitungseinrichtung (25) dazu eingerichtet ist, den Zeiger (21) in Abhängigkeit von Veränderungen der Position der Instrumentenspitze (19) zu bewegen.

8. Einrichtung nach Anspruch 7, **dadurch gekennzeichnet, dass** die Bildverarbeitungseinrichtung (25) darauf eingerichtet ist, die Bewegung des Zeigers (21) mit einem Vergrößerungsfaktor oder mit einem Verkleinerungsfaktor an die Bewegung der Instrumentenspitze (19) zu koppeln.

9. Einrichtung nach einem der vorstehenden Ansprüche,
**dadurch gekennzeichnet, dass** die Einrichtung (10) eine Quittiereinrichtung (27) aufweist, die dazu eingerichtet ist, ein Quittiersignal (q) zu erzeugen, und die mit der Erfassungseinrichtung (26) verbunden ist, um das Quittiersignal (q) an diese zu übermitteln.

10. Einrichtung nach Anspruch 5 und 9, **dadurch gekennzeichnet, dass** die Erfassungseinrichtung (26) darauf eingerichtet ist, bei Empfang eines Quittiersignals (q) Einstellparameter an das Gerät (15) zu übermitteln, die für dasjenigen Feld (22, 23, 24) hinterlegt sind, auf dem der Zeiger (21) positioniert ist.

11. Einrichtung nach Anspruch 9 oder 10, **dadurch gekennzeichnet, dass** die Bildverarbeitungseinrichtung (25) dazu eingerichtet ist, das Menü (20) in Abhängigkeit von einem Aktivierungsbefehl (a) ein- und auszublenden.

12. Einrichtung nach Anspruch 11, **dadurch gekennzeichnet, dass** die Quittiereinrichtung (27) dazu eingerichtet ist, den Aktivierungsbefehl (a) zu erzeugen.

13. Einrichtung nach Anspruch 12, **dadurch gekennzeichnet, dass** die Quittiereinrichtung (27) einen Hand-oder Fußtaster umfasst.

14. Einrichtung nach Anspruch 12, **dadurch gekennzeichnet, dass** die Quittiereinrichtung (27) dazu eingerichtet ist, vorgegebene Bewegungen der Instrumentenspitze (19) zu erfassen und in Abhängigkeit von erfassten Bewegungen Quittiersignale (q) und/oder Aktivierungssignale (a) zu erzeugen.

15. Verfahren zur Einstellung eines Geräts (15) zur Speisung eines chirurgischen Instruments (11), wobei bei dem Verfahren:
mit einer Bildaufnahmeeinrichtung (14) ein Bild (B) von einem Operationsort (12) aufgenommen und an eine Bildverarbeitungseinrichtung (25) übertragen wird,
ein zur Einwirkung auf den Patienten am Operationsort (12) geeignetes Instrument (11) mit seiner Instrumentenspitze (19) an dem Operationsort (12) positioniert wird,
mit einer Bildwiedergabeeinrichtung (18):
a) sowohl von dem Operationsort (12) als auch von der Instrumentenspitze (19) ein Bild (B) aufgenommen wird,
b) außerhalb des Zentrums des aufgenommenen Bilds (B) wenigstens ein Menü (20) angezeigt wird und
c) auf dem Menü (20) ein Zeiger (21) angezeigt wird, der mit dem Instrument (11) bewegungsgekoppelt ist.
